# FASCICULE DE BREVET EUROPEEN

(11) **EP 0 429 591 B1**
(45) Date de publication et mention de la délivrance du brevet: **17.08.1994**
(21) Numéro de dépôt: 90908454.3
(22) Date de dépôt: 12.06.1990
(51) Int. Cl.: F04B 43/04, F04B 21/02, F04B 43/00

(54) **MICROPOMPE PERFECTIONNEE**
MIKROPUMPE
IMPROVED MICRO-PUMP

(30) Priorité: 14.06.1989 CH 2241/89; 07.08.1989 FR 8910699
(43) Date de publication de la demande: 05.06.1991
(73) Titulaire: WESTONBRIDGE INTERNATIONAL LIMITED, Ballsbridge, Dublin 4 (IE)
(72) Inventeur: VAN LINTEL, Harald, T., G., CH-1920 Martigny (CH)
(74) Mandataire: Leger, Jean-François
(86) Numéro de dépôt international: CH9000145
(87) Numéro de publication internationale: WO9015929

(56) Documents cités:
- EP-A- 134 614
- DE-A- 2 639 992
- FR-A- 2 127 774
- GB-A- 2 077 367
- Sensors and Actuators, vol. 15, no. 2, octobre 1988, Lausanne, CH, pages 154-167, van Lintel: "A piezoelectric micropump based on micromachining of silicon"

## Description

La présente invention concerne une micropompe comportant une première plaquette en matière susceptible d'être usinée par des techniques photolithographiques de manière a définir avec au moins une seconde plaquette de support accolée face à face à la première plaquette, une chambre de pompage, un premier clapet, de type ant-retour à travers lequel ladite chambre de pompage peut sélectivement communiquer avec une entrée de la pompe et un second clapet de type à membrane à travers lequel ladite chambre de pompage peut sélectivement communiquer avec une sortie de la pompe, des moyens étant prévus pour provoquer une variation périodique de volume de ladite chambre de pompage, le second clapet étant commandé par la pression du fluide à expulser de la pompe.

De telles pompes peuvent être utilisées notamment pour l'administration in situ de médicaments, la miniaturisation de la pompe permettant à un malade de la porter sur soi, voire éventuellement de recevoir une pompe directement implantée dans le corps. Par ailleurs, de telles pompes permettent un dosage précis de faibles quantités de fluide à injecter.

On connait des micropompes de ce type par un article intitulé "A piezoelectric micropump based on micromachining of silicon" et paru dans "Sensors and Actuators" No 15 (1988), pages 153 à 167, dans lequel H. Van Lintel et al. donnent une description de deux formes de réalisaiton d'une micropompe, comportant chacune un empilement de trois plaquettes, c'est-à-dire une plaquette en silicium usinée disposée entre deux plaquettes en verre.

La plaquette en silicium définit une chambre de pompage avec l'une des plaquettes en verre dont la partie coïncidant avec cette chambre peut être déformée par un élément moteur qui est, en l'occurence, un cristal piézoélectrique. Ce dernier comporte des électrodes qui, lorsqu'elles sont raccordées à une source de tension alternative, provoquent la déformation du cristal et par suite de la plaquette en verre celle-ci, à son tour, faisant varier le volume de la chambre de pompage.

La chambre de pompage est raccodée de part et d'autre respectivement à des clapets anti-retour usinés dans le silicium et dont le siège est constitué par l'autre plaquette en verre.

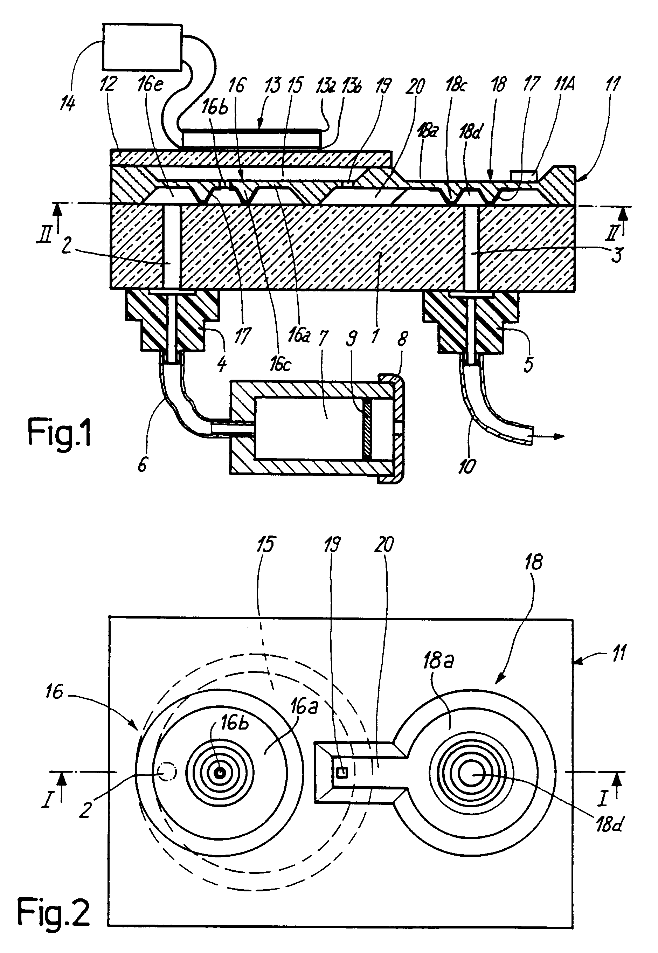

Une analyse du fonctionnement de la pompe selon la première forme de réalisation (figure 1a) décrite dans l'article précité montre que cette pompe délivre un débit de fluide qui est fortement dépendant de la pression de sortie sur toute la plage de fonctionnement. En effet, on a constaté que cette relation débit-pression est pratiquement linéaire, le débit étant d'autant plus faible que la pression est plus élevée.

En d'autres termes, une telle pompe est inutilisable pour les applications médicales précitées dans lesquelles, au contraire, le débit de sortie de la pompe doit être indépendant de la pression, tout au moins dans la plage normale de fonctionnement de la pompe.

C'est pourquoi les auteurs proposent dans ce même article (seconde forme de réalisation représentée à la figure 1b) d'adjoindre à l'ensemble que l'on vient de décrire un clapet régulateur interposé entre le second clapet qui fait suite à la chambre de pompage, et la sortie de la pompe. Ce clapet isole la pompe de la sortie lorsqu'il est fermé.

Par ailleurs, le clapet régulateur disposant d'une certaine précontrainte à la fermeture, la pression de sortie ne peut ouvrir le clapet qu'à partir d'une certaine valeur. Il en résulte ainsi dans la plage utile de fonctionnement de la pompe, une quasi indépendance du débit par rapport à la pression de sortie, c'est-à-dire tant que le clapet régulateur n'est pas maintenu ouvert par la pression de sortie.

Si donc grâce à cette seconde construction on obtient un diagramme débit-pression favorable, il faut cependant observer qu'une pompe réalisée de cette façon présente encore des inconvénients. Le clapet régulateur augmente l'encombrement de la pompe, car il doit être réalisé dans l'épaisseur de la plaquette de silicium et occupe ainsi de la surface supplémentaire. Il en résulte par conséquent une augmentation du prix de revient de la pompe.

Il est également à noter que le clapet régulateur augmente la complexité de la pompe et ainsi les risques de mauvais fonctionnement ou de rebut à la fabrication.

Citons encore le brevet FR 2 127 774 qui décrit une pompe à membrane du type classique ne faisant pas appel à des techniques de micro-usinage de plaquettes de silicum par photolithographie, et qui comprend une chambre de pompage située entre un clapet d'entrée et un clapet de sortie. Le canal de sortie de cette pompe est en outre raccordé en une chambre située du côté opposé de la membrane par rapport à la chambre de pompage. Le débit de fluide de cette pompe est donc fortement dépendant de la pression de sortie.

La présente invention a pour but de fournir une micropompe du type indiqué ci-dessus, qui permette d'éviter les inconvénients précités tout en présentant une caractéristique du débit en fonction de la pression de sortie favorable dans la plage de fonctionnement utile de la pompe.

L'invention est caractérisée, à cet effet, en ce que ladite sortie est en communication directe avec un volume isolé de ladite chambre de pompage par ledit second clapet et situé du même côté de ce clapet que le canal par lequel ce clapet communique avec la chambre de pompage, de manière que les pressions régnant respectivement dans cette chambre de pompage et ce volume agissent dans le sens de l'ouverture sur ledit second clapet, et en ce que ledit second clapet est en communication ouverte avec ledit premier clapet par l'intermédiaire de ladite chambre de pompage, de manière que, lors de la phase de refoulement de la pompe, cette chambre communique directement avec ladite sortie, à travers ledit second clapet en position ouverte.

Grâce à ces caractéristiques, ledit second clapet non seulement assure une régulation du débit de manière à rendre ce dernier quasiment indépendant de la pression dans la sortie de la pompe, sur toute sa plage de fonctionnement normal, mais encore il agit comme l'organe fermant la chambre de pompage au cours de la phase d'aspiration de la pompe.

D'autres caractéristiques et avantages de l'invention apparaîtront au cours de la description qui va suivre de plusieurs modes de réalisation de la micropompe suivant l'invention, description faisant référence aux dessins annexés sur lesquels :
- la figure 1 est une vue en coupe schématique d'une micropompe selon l'invention;
- la figure 2 montre une vue de dessous de la plaquette intermédiaire de la pompe représentée à la figure 1;
- la figure 3 est une vue montrant la face inférieure de la plaquette intermédiaire d'une micropompe construite selon un second mode de réalisation de l'invention, la vue étant prise selon la ligne III-III de la figure 4;
- les figures 4 et 5 sont des vues en coupe prises respectivement selon les lignes IV-IV et V-V de la figure 3;
- la figure 6 est une vue en coupe d'une micropompe construite selon un troisième mode de réalisation de l'invention;
- la figure 7 est une vue de la face inférieure de la plaquette intermédiaire de la pompe représentée à la figure 6, cette vue étant prise selon la ligne VII-VII de la figure 6;
- la figure 8 est une vue de la face inférieure d'une plaquette intermédiaire appartenant à une micropompe construite selon un quatrième mode de réalisation de l'invention;
- les figures 9 et 10 sont des vues en coupe prises respectivement selon les lignes IX-IX et X-X de la figure 8;
- la figure 11 montre une vue partielle en coupe d'un cinquième mode de réalisation de l'invention; - la figure 12 est une vue de dessus partielle de la micropompe représentée à la figure 11; et
- la figure 13 est un graphique représentant la caractéristique du débit en fonction de la pression mesurée sur une micropompe construite selon l'invention, la pression d'entrée étant égale à zéro.

On va tout d'abord se référer aux figures 1 et 2 qui représentent un premier mode de réalisation de la micropompe suivant l'invention.

Il est à noter que par souci de clarté, les épaisseurs des diverses plaquettes composant la micropompe ont été fortement exagérées sur les dessins.

La micropompe des figures 1 et 2 comporte une plaquette de base 1 en verre par exemple, qui est percée de deux canaux 2 et 3 formant respectivement la conduite d'aspiration et la conduite de refoulement de la pompe. Ces canaux 2 et 3 communiquent avec des raccords 4 et 5 respectifs.

Le raccord 4 est branché sur un tuyau 6 lui-même raccordé à un réservoir 7 dans lequel se trouve la substance liquide à pomper. Le réservoir est obturé par un capuchon percé, un piston mobile isolant le volume utile du réservoir 7 de l'extérieur. Ce réservoir peut contenir un médicament par exemple, au cas où la pompe est utilisée pour l'injection de ce médicament dans le corps humain avec un dosage précis. Dans cette application, la micropompe peut être portée sur le corps du patient, voire être implantée.

Le raccord de refoulement 5 peut être branché à une aiguille d'injection (non représentée) qui y est raccordée par un tuyau 10.

L'utilisation de cette manière de la micropompe de l'invention est particulièrement appropriée pour le traitement à l'aide de peptides de certaines formes de cancers dont la médication est réalisée, de préférence, par un dosage précis et répété à des intervalles réguliers de petites quantités du médicament. Une autre application peut être envisagée pour le traitement des diabétiques devant recevoir périodiquement de faibles doses de médicament au cours de la journée, le dosage pouvant être déterminé par exemple par des moyens connus en soi, mesurant le taux de sucre dans le sang et commandant automatiquement la pompe pour qu'une dose d'insuline appropriée puisse être injectée.

Une plaquette 11 en silicium ou en un autre matériau usinable par gravure à l'aide des techniques photolithographiques, est accolée à la plaquette en verre 1. Cette plaquette en silicium est surmontée elle-même d'une plaquette de fermeture en verre 12 dont l'épaisseur est telle qu'elle puisse être déformée par un élément de commande 13 qui, dans l'application de l'invention décrite ici est une pastille piézo-électrique pourvue d'électrodes 13a et 13b branchées sur un générateur 14 de tension alternative. Cette pastille peut être celle fabriquée par la Société Philips sous la dénomination PXE-52 et elle peut être collée sur la plaquette 12 au moyen d'une colle appropriée.

A titre d'exemple, la plaquette intermédiaire 11 en silicium peut avoir une orientation cristalline 〈100〉, afin de bien se prêter à la gravure et d'avoir la solidité nécessaire. Les plaquettes 1 et 12 sont de préférence soigneusement polies.

Les plaquettes 11 et 12 délimitent ensemble tout d'abord une chambre de pompage 15 (voir aussi la figure 2), de forme circulaire par exemple, cette chambre se trouvant située en-dessous d'une zone de la plaquette 12 qui est déformable par l'élément de commande 13.

Entre la canalisation d'aspiration 2 et la chambre de pompage 15 est interposé un premier clapet 16 du type anti-retour usiné dans la plaquette en silicium 11. Ce clapet est situé sous la chambre de pompage et comporte une membrane 16a de forme générale circulaire et percée en son centre d'un orifice de passage 16b qui, dans le mode de réalisation représenté, est de forme carrée. Du côté de la canalisation 2, le clapet 16 comporte une nervure 16c de forme annulaire et de section à peu près triangulaire. Cette nervure 16c entoure l'orifice 16b et est revêtue d'une fine couche d'oxyde 17 obtenue également par des techniques de photolithographie. Cette couche d'oxyde provoque une surépaisseur qui confère à la membrane 16a une certaine précontrainte ou prétension lorsque le sommet de la nervure 16c est appliqué contre la plaquette en verre 1, cette dernière servant ainsi de siège au clapet 16.

La canalisation de refoulement 3 de la pompe communique avec la chambre de pompage 15 par l'intermédiaire d'un clapet 18 dont la construction est identique à celle du clapet 16, à ceci près cependant que par différence de dimension de la couche 17 par rapport à celle du clapet 16, la précontrainte assurée par cette couche d'oxyde 17 peut être différente de celle utilisée pour le clapet 16. En outre, on voit sur la figure 1 que ce clapet est dépourvu d'un orifice central tel que l'orifice 16b du clapet 16.

On notera que la chambre de pompage communique avec le clapet 18 par l'intermédiaire d'un orifice 19 et d'un passage 20 tous deux usinés dans la plaquette en silicium 11.

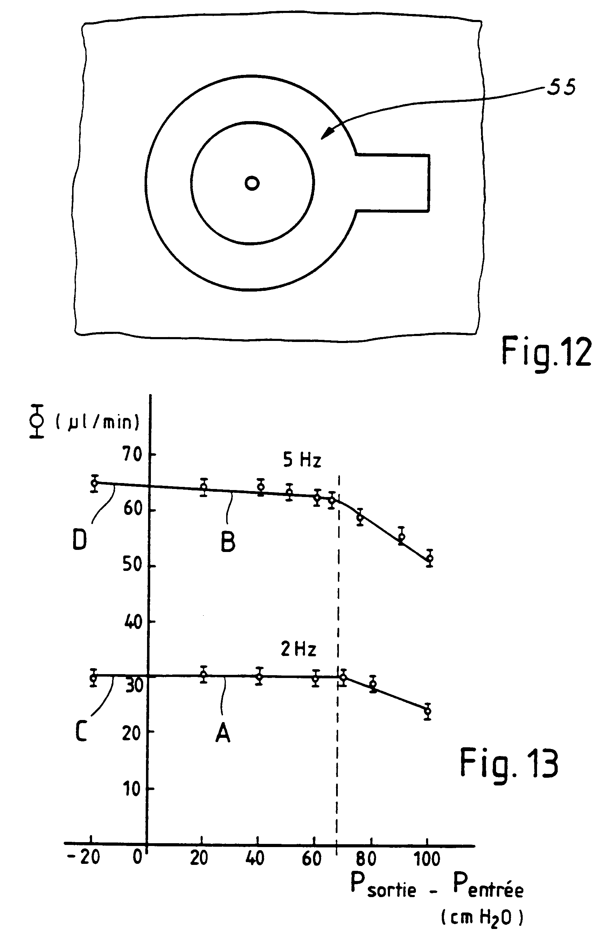

Le clapet 18 comporte donc une membrane 18a partiellement revêtue et une nervure annulaire 18c revêtue d'une couche d'oxyde 17 et délimite au-dessus de la canalisation 3 un volume 18d dans lequel règne la pression de sortie. La couche d'oxyde de la membrane 18a crée des forces de cisaillement dans celle-ci, qui induisent un bombement de cette membrane (la couche d'oxyde est du côté convexe de la membrane). Ceci provoque une prétension supplémentaire du clapet dans le sens de la fermeture, par rapport à la prétension induite par la couche d'oxyde recouvrant la nervure 18c.Lorsque le clapet 18 est ouvert, ce volume est en communication directe avec le clapet d'aspiration 16 à travers la chambre de pompage 15 avec comme résultat qu'un minimum de résistance à l'écoulement est imposé au fluide refoulé de la pompe lors de la phase de refoulement. En outre, lorsque le clapet 18 est fermé, la pression de sortie n'agit que sur une faible surface de la membrane 18a comparée à la surface nettement plus importante sur laquelle peut agir la pression régnant dans la chambre de pompage. Ceci a pour effet une régulation du débit de sortie qui devient pratiquement indépendant de la pression de sortie (voir figure 13), cet effet étant provoqué par la prétension assurée par la couche d'oxyde 17.

Pour fixer les idées, les épaisseurs des plaquettes 1, 11 et 12 peuvent respectivement être d'environ 1 mm, 0,3 mm et 0,2 mm pour une dimension en surface des plaquettes de l'ordre de 15 par 20 mm.

Par ailleurs, les plaquettes peuvent être fixées les unes aux autres par diverses techniques de liaison connues telles que le collage ou la technique connue sous le nom de soudure anodique, par exemple.

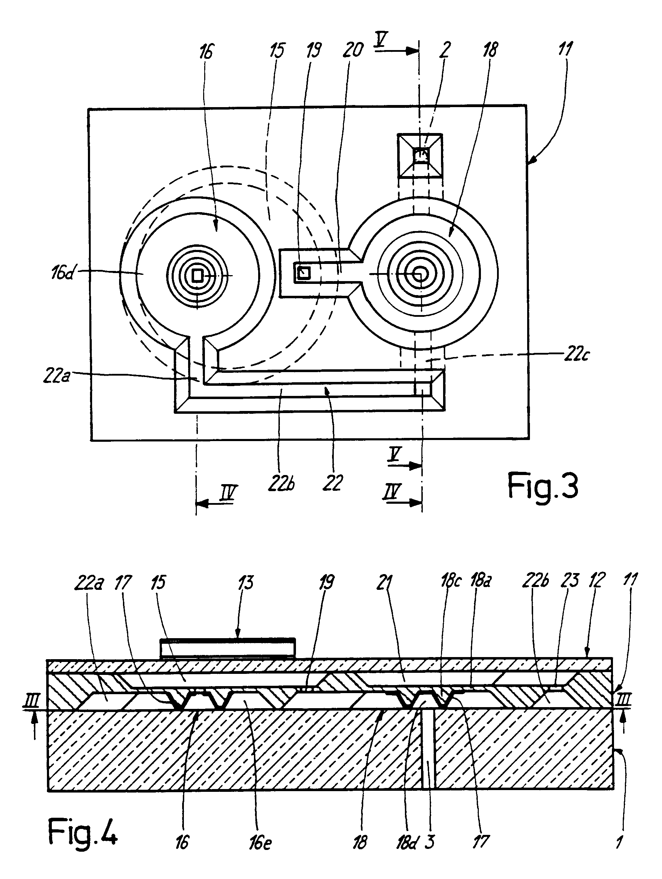

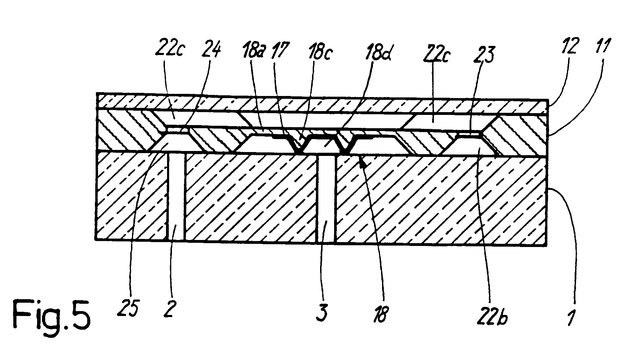

Les figures 3 à 5 montrent un second mode de réalisation de la micropompe suivant l'invention qui est pour l'essentiel d'une construction identique à celle de la micropompe représentée aux figures 1 et 2. Les éléments identiques ont donc été repérés par les mêmes références que précédemment. Cependant, elle en diffère en ce que la chambre annulaire 16e (figure 4) qui entoure la nervure annulaire 16c du clapet d'aspiration 16 est raccordée non seulement à la canalisation d'aspiration 2, mais également à une chambre de compensation 21 définie dans la plaquette 11 au-dessus du clapet 18, et fermée par la plaquette de fermeture 12, celle-ci recouvrant ici la totalité de la surface de la pompe. Ce raccordement est réalisé par l'intermédiaire d'un canal de communication 22 usiné dans le silicium et formé de trois branches 22a, 22b et 22c disposées à angle droit dans la plaquette 11. Il est à noter que la branche 22c de cette canalisation ne se trouve pas au même niveau que les deux autres branches 22a et 22b, les branches 22b et 22c étant en communication l'une avec l'autre à travers un orifice de communication 23 pratiqué dans la plaquette 11. Par ailleurs, la branche 22c (figure 5) est en communication avec la canalisation d'aspiration 2 par l'intermédiaire d'un orifice de communication 24 qui relie cette branche à une petite cavité 25 creusée dans la plaquette de silicium 11 juste au-dessus de la canalisation d'aspiration 2.

La canalisation 22 est destinée à faire communiquer le canal 2 de la pompe, avec la chambre 21 ménagée, au-dessus de la membrane 18a du clapet de refoulement 18 de manière à maintenir celui-ci fermé si une surpression se présente à l'entrée de la pompe. Cet agencement agit donc comme sécurité contre les surpressions.

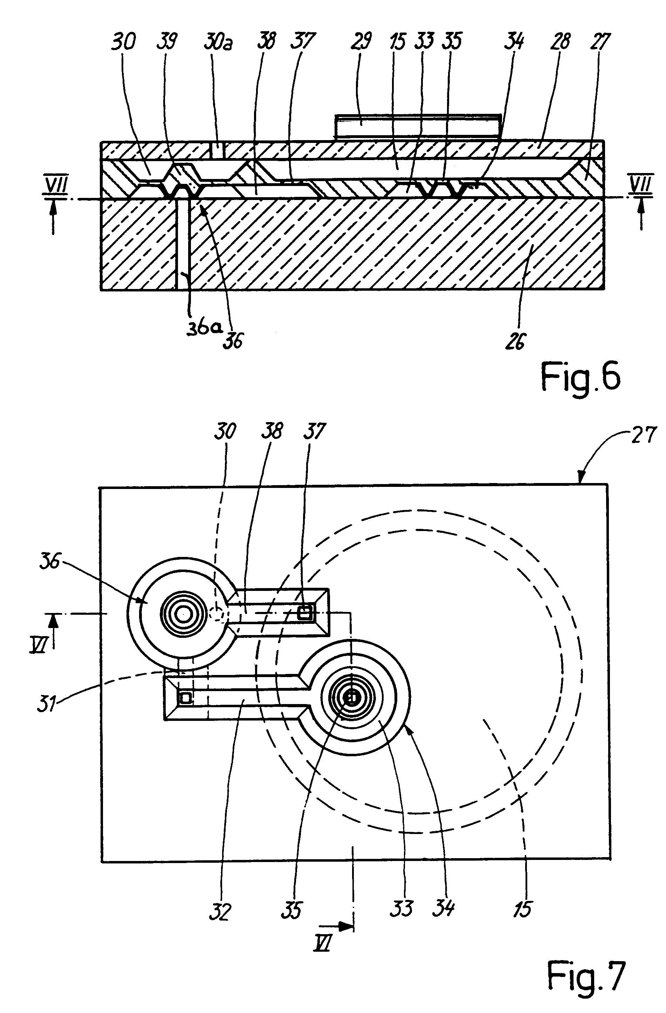

Les figures 6 et 7 auxquelles on va se référer maintenant représentent un troisième mode de réalisation de la micropompe suivant l'invention. Dans ce cas, le principe de la construction, précédemment décrit, reste le même cependant que la chambre de pompage 15 est disposée de façon asymétrique par rapport aux clapets d'aspiration et de refoulement.

Cette pompe est composée également de trois plaquettes, à savoir une plaquette de support 26, en verre par exemple, une plaquette 27, en silicium par exemple ou en une autre matière appropriée, et une plaquette de fermeture 28 en verre par exemple qui est déformable, dans une zone située au-dessus de la chambre de pompage 15, à l'aide d'une pastille piézo-électrique 29 ou d'un autre élément de commande approprié.

La chambre de pompage 15 est délimitée par la plaquette 27 et la plaquette 28, ces deux plaquettes définissant également une chambre d'entrée 30 (visible sur la figure 6 uniquement) dans laquelle débouche un orifice d'entrée 30a qui est pratiqué dans la plaquette 28.

La chambre 30 communique avec un canal 31 (qui n'est visible que sur la figure 7) situé dans la partie supérieure de la plaquette 27 et cette canalisation 31 communique avec une seconde canalisation 32 qui est ménagée dans la plaquette 27 du côté de la plaquette 26. La canalisation 32 débouche dans une chambre annulaire 33 d'un clapet d'aspiration 34 dont la construction est identique à celle du clapet 16 précédemment décrit. Ce clapet d'aspiration communique avec la chambre 15 à travers un orifice central 35.

La chambre 15 communique également avec un clapet de refoulement 36 par l'intermédiaire d'un orifice 37 et d'une canalisation 38 ménagés tous deux dans la plaquette 27. Par ailleurs, le clapet 36 de refoulement qui est destiné à obturer un orifice de sortie 36a (figure 6) est construit de la même manière que le clapet de refoulement des précédents modes de réalisation à ceci près qu'il comporte un bossage 39 du côté de sa membrane opposée à la nervure d'obturation de clapet. Ce bossage 39, qui se trouve au centre de la membrane, est destiné à limiter l'amplitude du mouvement de celle-ci grâce à l'effet de butée qu'il peut exercer sur la plaquette 28 au cas où la pression de sortie dépasse une valeur admissible prédéterminée.

Dans l'agencement que l'on vient de décrire, l'orifice d'entrée 30a débouche dans la chambre 30 qui est située au-dessus du clapet de refoulement 36 et fait donc office de chambre de compensation, comme la chambre 21 du précédent mode de réalisation. Par conséquent, cette construction permet également d'obtenir une sécurité contre les surpressions.

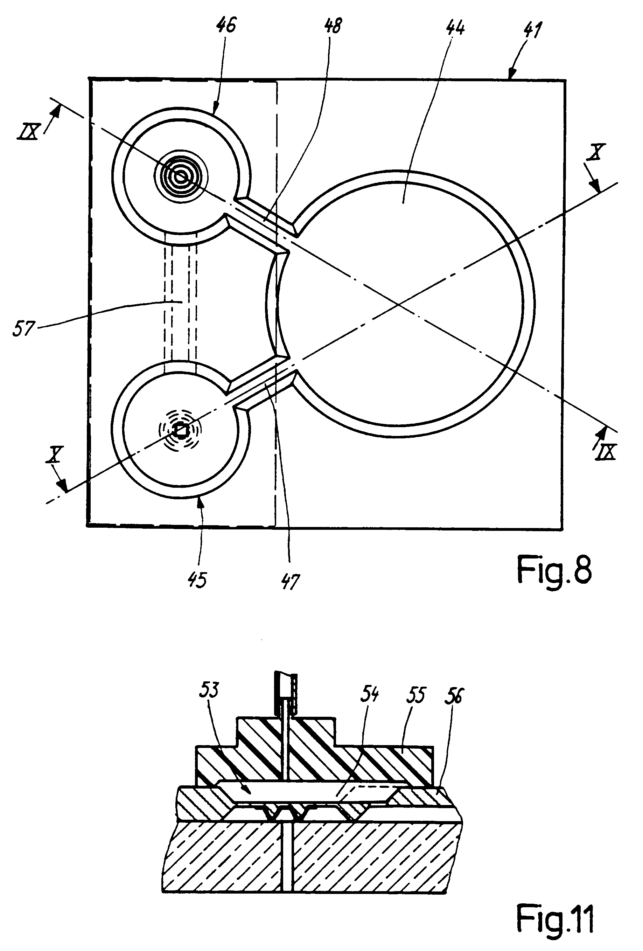

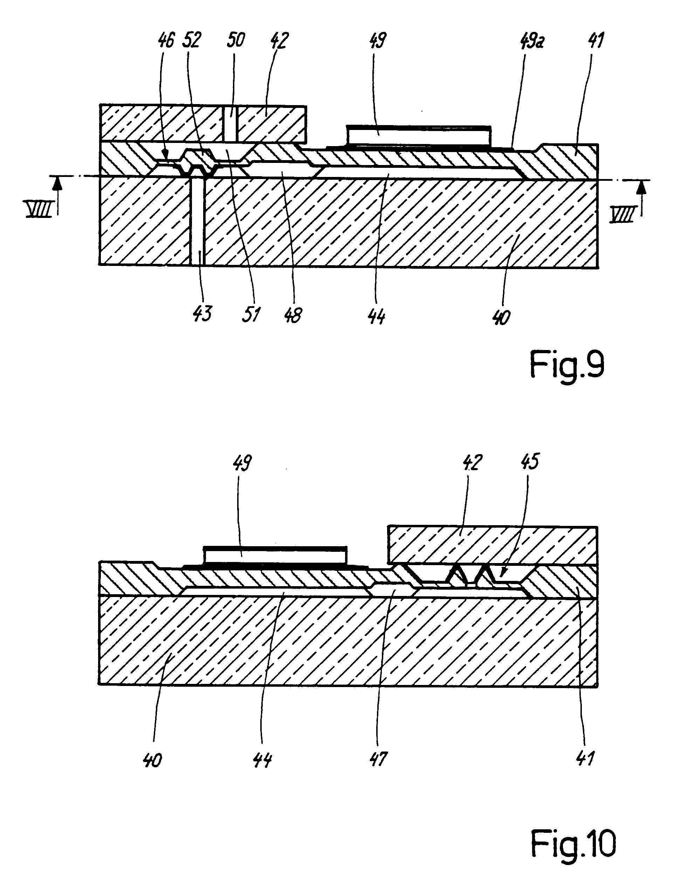

On va se référer maintenant aux figures 8 à 10 qui représentent un quatrième mode de réalisation de l'invention.

Dans ce cas, la micropompe comporte également trois plaquettes 40, 41 et 42. La plaquette 40 est réalisée en verre par exemple et comporte une canalisation de refoulement 43. La plaquette 41 est réalisée en silicium ou une autre matière appropriée et conformée, à l'aide de procédés photolithographiques, de manière à définir une chambre de pompage 44, un clapet d'aspiration 45 et un clapet de refoulement 46 communiquant respectivement avec la chambre de pompage 44 par l'intermédiaire de canalisations 47 et 48.

Dans le présent mode de réalisation, un élément de commande tel qu'une pastille piézo-électrique 49 est placé directement sur la plaquette 41 en silicium dans la zone qui coïncide avec la chambre de pompage 44 si bien que pour obtenir l'effet de pompage, c'est cette plaquette 41 qui est déformée pour modifier le volume de la chambre de pompage. Il est en outre souhaitable de prévoir entre la plaquette 41 et le cristal 49 une mince couche d'oxyde de silicium 49a afin d'isoler l'électrode correspondante de la pastille par rapport à cette plaquette.

La plaquette 42 ne recouvre que partiellement la plaquette 41 et elle comporte un orifice d'aspiration 50 qui débouche dans une chambre annulaire de compensation 51 prévue au-dessus du clapet de refoulement 46. On voit que ce clapet est pourvu d'un bossage 52 permettant de limiter l'amplitude de la membrane de ce clapet, le bossage pouvant venir s'appuyer contre la face inférieure de la plaquette 42 au cas où la pression de sortie sous le clapet deviendrait excessive.

On notera également que les positions des clapets d'aspiration et de refoulement 45 et 46 sont inversées, le clapet d'aspiration ayant son siège constitué par la plaquette 42, tandis que le siège du clapet 46 est constitué, comme dans les autres modes de réalisation, par la plaquette 40. Cette disposition n'a aucune influence particulière sur le fonctionnement de la pompe.

On a représenté sur la figure 11 un autre mode de réalisation de l'invention dans lequel, au-dessus du clapet de refoulement 53, est prévue une chambre 54 qui est obturée par un organe de connexion 55 réalisé en matière plastique par exemple et collé sur la plaquette en silicium 56. Ainsi, la chambre 54 qui est en fait en communication avec l'aspiration de la pompe est isolée totalement de l'atmosphère extérieure. Cette construction a l'avantage de permettre d'éviter l'utilisation d'une plaquette de fermeture particulière.

Tant dans la pompe suivant les figures 8 à 10 (canalisation 57) que dans celle de la figure 11 (chambre 54), les clapets de refoulement sont connectés à l'entrée de la pompe de manière à assurer dans ces cas également, la sécurité contre les surpressions.

On va maintenant examiner le fonctionnement de la micropompe suivant l'invention en se reportant plus particulièrement au diagramme de la figure 13 qui représente le débit en fonction de la pression dans la canalisation de refoulement.

On notera que le fonctionnement est le même quels que soient les modes de réalisation décrits ci-dessus. Pour plus de commodité, la description ci-après se reporte donc uniquement au mode de réalisation des figures 1 et 2.

Lorsque aucune tension électrique n'est appliquée à la pastille piézo-électrique 13, les clapets d'aspiration 16 et de refoulement 18 sont en position fermée. Lorsqu'une tension électrique est appliquée, la pastille piézo-électrique 13 se déforme faisant fléchir la plaquette 12 vers l'intérieur. Il se produit alors une augmentation de pression dans la chambre de pompage 15 qui provoque l'ouverture du clapet de refoulement 18 dès que la force agissant sur la membrane en raison de la pression dans la chambre 15 est supérieure à la différence entre la force créée par la prétension du clapet 18, assurée par la couche d'oxyde de silicium 17 et la force due à la pression dans le canal de sortie 3. Le fluide contenu dans la chambre de pompage est alors refoulé vers le canal de sortie 3 par le déplacement de la zone déformable de la plaquette 12. Pendant cette phase, le clapet d'aspiration 16 est maintenu fermé par la pression régnant dans la chambre de pompage 15. Le fluide s'écoule sans rencontrer de résistance notable, du fait que la chambre de pompage 15 est alors directement en communication avec le canal de sortie 3.

Au contraire, lorsqu'on supprime la tension électrique, la pastille piézo-électrique 13 reprend sa forme initiale, voire se déforme dans l'autre sens si bien que la pression dans la chambre de pompage 15 diminue. Ceci provoque la fermeture du clapet de refoulement 18, dès que la force due à la pression dans la chambre de pompage 15 est inférieure à la différence entre la force créée par la prétension du clapet et la force due à la pression dans le canal de sortie 3. L'ouverture du clapet d'aspiration 16 a lieu dès que la somme de la force due à la pression dans la chambre de pompage et de la force créée par la prétension du clapet 16 est inférieure à la force due à la pression dans le canal d'entrée 2. Il y a alors aspiration de fluide dans la chambre de pompage 15 par le canal d'entrée 2 par suite du déplacement de la zone déformable de la plaquette 12.

En choisissant un grand rapport du diamètre de la membrane du clapet de refoulement 18 et de son siège, la pression dans le canal de refoulement 3 influence peu la pression de la chambre de pompage, nécessaire pour ouvrir le clapet de refoulement. Par conséquent, en choisissant de manière judicieuse ce rapport, ainsi que la fréquence de commande de la pastille piézo-électrique, on peut obtenir que la pression de sortie n'influence que très peu le débit de sortie. On peut ainsi déterminer des courbes débit/pression de sortie telles que celles qui sont représentées sur la figure 13. Sur cette figure, la courbe A a été obtenue en appliquant sur la pastille piézo-électrique d'une micropompe selon les figures 3 à 5, ayant environ les dimensions indiquées ci-dessus, une tension à 2 Hz, le débit de sortie restant pratiquement constant à une valeur de 30 µl/min (choix le plus favorable). Si on commande la pastille piézo-électrique avec une tension à 5 Hz, ce débit est porté à 64 µl/min, environ. Ainsi que l'on peut le voir sur les courbes de la figure 13, le choix d'une prétension donnée sur la membrane du clapet de refoulement maintient le débit constant aux valeurs indiquées alors que la pression de sortie peut varier de 0 à 70 cm H₂O. D'ailleurs, les courbes montrent également que le débit reste constant même pour des valeurs négatives de la différence entre la pression de sortie et la pression d'entrée (parties C et D des courbes respectives). Ce cas peut se produire par exemple en présence d'une surpression à l'entrée de la pompe.

On notera que la micropompe, quel que soit son mode de réalisation décrit ci-dessus, est compacte, et simple, présente une résistance faible à l'écoulement et permet de déterminer avec une très bonne approximation un débit constant en fonction de la pression de sortie.

Dans tous les modes de réalisation, sauf celui des figures 1 et 2, la pompe a été conçue pour assurer une sécurité contre les surpressions grâce au fait que l'entrée de la pompe est en communication avec une chambre située au-dessus du clapet de refoulement. Par conséquent, si une telle surpression se présente, elle agit sur le clapet de refoulement dans le sens de la fermeture isolant ainsi le canal de refoulement de la chambre de pompage. Cette propriété de la pompe peut être importante lorsqu'il s'agit d'une pompe portée par un patient qui porte également un réservoir souple. Si ce dernier comprime alors le réservoir (par exemple par ce qu'il se heurte à un obstacle), la surpression dans l'aspiration de la pompe ne provoque aucun débit côté refoulement de celle-ci.

En outre, grâce à la mise en communication de la chambre de compensation avec l'entrée de la pompe, une variation de la pression d'entrée n'a, sur la plage normale de fonctionnement de la pompe, que très peu d'influence sur le débit de sortie.

Il est à noter que la mise en communication peut être réalisée également par une connexion extérieure à la pompe. Par exemple, la pompe des figures 1 et 2 pourrait être pourvue d'un tuyau reliant l'entrée à la sortie.

Enfin, la figure 1 montre que l'on peut associer à la plaquette en silicium, un élément de contrôle de fonctionnement, par exemple, sous la forme d'une jauge de contrainte 11A dont la variation de résistance pourra être mesurée pour témoigner du bon fonctionnement de la pompe ou de la rupture de la membrane sur laquelle la jauge est collée. Bien entendu, un ou plusieurs de ces éléments de contrôle peuvent être prévus dans tous les modes de réalisation décrits.

## Revendications

1. Micropompe comportant une première plaquette (11; 27; 41; 56) en matière susceptible d'être usinée par des techniques photolithographiques de manière à définir avec au moins une seconde plaquette de support (1; 26; 40) accolée face à face à la première plaquette, une chambre de pompage (15;44), un premier clapet de type anti-retour (16; 33; 45) à travers lequel ladite chambre de pompage (15; 44) peut sélectivement communiquer avec une entrée (2; 30; 50) de la pompe et un second clapet de type à membrane (18; 36; 46) à travers lequel ladite chambre de pompage peut sélectivement communiquer avec une sortie (3; 36a; 43) de la pompe, des moyens (13; 14; 29; 49) étant prévus pour provoquer une variation périodique de volume de ladite chambre de pompage (15; 44), le second clapet étant commandé par la pression du fluide à expulser de la pompe, caractérisé en ce que ladite sortie (3; 36a; 43) est en communication directe avec un volume (18d) isolé de ladite chambre de pompage (15; 44) par ledit second clapet (18; 36; 46) et situé du même côté de ce clapet que le canal (19; 20) par lequel ce clapet communique avec la chambre de pompage, de manière que les pressions régnant respectivement dans cette chambre de pompage et le canal de sortie agissent dans le sens de l'ouverture sur ledit second clapet, et en ce que ledit second clapet (18; 36; 46) est en communication ouverte avec ledit premier clapet (16; 33; 45) par l'intermédiaire de ladite chambre de pompage (15; 44), de manière que, lors de la phase de refoulement de la pompe, cette chambre communique directement avec ladite sortie (3; 36a; 43), à travers ledit second clapet en position ouverte.

2. Micropompe suivant la revendication 1, caractérisée en ce qu'elle comprend une troisième plaquette (12; 28; 42; 55) recouvrant ladite première plaquette (11; 27; 41) au moins sur une partie de sa surface et délimitant avec elle au moins une chambre (15, 21; 51; 54) destinée à former une cavité active de la pompe.

3. Micropompe suivant la revendication 2, caractérisée en ce que lesdits moyens prévus pour provoquer une variation périodique de volume de la chambre de pompage comprennent une pastille piézoélectrique (13; 29) fixée sur ladite troisième plaquette (12; 28) dans une zone qui coïncide avec ladite chambre de pompage (15).

4. Micropompe suivant la revendication 2, caractérisée en ce que lesdits moyens prévus pour provoquer une variation périodique de volume de la chambre de pompage comprennent une pastille piézo-électrique (49), en ce que ladite chambre de pompage (44) est délimitée par lesdites première (41) et seconde (40) plaquettes et en ce que ladite pastille piézo-électrique est fixée sur ladite première plaquette dans une zone à découvert de celle-ci coïncidant avec ladite chambre de pompage (44).

5. Micropompe suivant l'une quelconque des revendications 2 à 4, caractérisée en ce que ladite troisième plaquette (55) est agencée pour former également au moins l'un des raccords au moyen duquel la pompe peut être connectée à un circuit extérieur (figure 11).

6. Micrompompe suivant l'une quelconque des revendications 1 à 5, caractérisée en ce qu'un élément détecteur, tel qu'une jauge de contrainte (11A) est fixée sur ladite première plaquette (11) pour permettre la surveillance du bon fonctionnement de la pompe.

7. Micropompe suivant l'une quelconque des revendications précédentes, caractérisée en ce que ledit premier clapet (16; 33; 45) et/ou ledit second clapet (18; 36; 46) comporte une membrane (16a; 18a) prévue dans le plan de ladite première plaquette au centre de laquelle est prévue une nervure annulaire (16c; 18c) dont le sommet est destiné à venir s'appliquer contre une autre plaquette (1; 12; 26; 28; 40; 41) faisant office de siège de ce clapet, et en ce qu'au moins ladite nervure (16c; 18c) est recouverte d'une couche d'oxyde (17) sollicitant la membrane avec une prétension prédéterminée de manière que le clapet soit normalement fermé.

8. Micropompe suivant la revendication 7, caractérisée en ce que la membrane (18a) du second clapet est au moins partiellement recouverte d'une couche d'un matériau créant dans la membrane des forces de cisaillement induisant une prétension supplémentaire.

9. Micropompe suivant l'une quelconque des revendications 7 et 8, caractérisée en ce que du côté opposé à ladite nervure (16c), ladite membrane (16a) est pourvue d'un bossage (39; figure 6) s'étendant vers une autre plaquette (28) de la pompe et destinée à limiter l'excursion de la membrane (16) au cas où elle est soumise à une pression excessive.

10. Micrompompe suivant l'une quelconque des revendications 7 à 9, caractérisée en ce que les nervures (16c), respectivement prévues sur les membranes (16a) desdits clapets sont déposées sur des faces opposées de ces membranes (clapets 45 et 46, figures 8, 9 et 10).

11. Micropompe suivant l'une quelconque des revendications précédentes, caractérisée en ce que l'entrée (2; 30; 50) de la pompe est en communication avec une chambre de compensation (21; 30; 51; 54) aménagée au-dessus dudit second clapet (18; 36; 46) afin de solliciter celui-ci à la fermeture sous l'influence de la pression régnant à ladite entrée.

## Claims

1. A micropump including a first thin slab (11; 27; 41; 56) of a material which can be etched by photolithography techniques in such a manner as to define with at least a second thin support slab (1; 26; 40) bonded by one face to the first thin slab, a pumping chamber (15; 44), a first valve of the nonreturn type (16; 33; 45) through which said pumping chamber (15; 44) can communicate selectively with an inlet (2; 30; 50) of the pump and a second valve of the membrane type (18; 36; 46) through which said pumping chamber can communicate selectively with an outlet (3; 36a; 43) of the pump, means (13; 14; 29; 49) being provided for generating a periodical variation of the volume of said pumping chamber (15; 44), the second valve being controlled by the pressure of the fluid to be discharged by the pump, characterized in that said outlet (3; 36a; 43) is in direct communication with a volume (18d) isolated from said pumping chamber (15; 44) by said second valve (18; 36; 46) and located on the same side of this valve as the channel (19; 20) through which this valve communicates with the pumping chamber, so that the pressures prevailing respectively in this pumping chamber and in the outlet channel act in a direction to open said second valve, and in that said second valve (18; 36; 46) is in open communication with said first valve (16; 33, 45) through said pumping chamber (15; 44), so that during the discharge phase of the pump, this chamber communicates directly with said outlet (3; 36a; 43) through said second valve in its open position.

2. A micropump according to claim 1, characterized in that it includes a third thin slab (12; 28; 42; 55) covering said first thin slab (11; 27; 41) at least on one part of its surface while defining therewith at least one chamber (15, 21; 51; 54) designed for providing an active cavity of the pump.

3. A micropump according to claim 2, characterized in that said means provided for generating a periodical variation of the volume of the pumping chamber include a piezoelectric wafer (13; 29) affixed to said third thin slab (12; 28), in a zone which coincides with said pumping chamber (15),

4. A micropump according to claim 2, characterized in that said means provided for generating a peridiodical variation of the volume of the pumping chamber include a piezoelectric wafer (49), in that said pumping chamber (44) is defined by said first (41) and second (40) thin slabs and in that said piezoelectric wafer is affixed to said first thin slab in an exposed zone thereof coinciding with said pumping chamber (44).

5. A micropump according to any one of claims 2 to 4, characterized in that said third thin slab (55) is formed so as to provide also one of the connections by which the pump can be connected to an external circuit (figure 11).

6. A micropump according to any one of claims 1 to 5, characterized in that a detector member, such as a strain gauge (11A), is affixed to said first thin slab (11) for controlling that the pump functions correctly.

7. A micropump according to any one of the preceding claims, characterized in that said first valve (16; 33; 45) and/or said second valve (18; 36; 46) include(s) a membrane (16a; 18a) arranged in the plane of said first thin slab, at the center of which is provided an annular rib (16c; 18c), the ridge of which is designed for application against another thin slab (1; 12; 26; 28; 40; 41) acting as the seat of this valve, and in that at least said rib (16c; 18c) is coated with a layer of oxide (17) for biasing the membrane with a predetermined pretension in such a manner that the valve be normally closed.

8. A micropump according to claim 7, characterized in that the membrane (18a) of the second valve is at least partly coated with a material generating within the membrane shearing forces inducing an additional pretension.

9. A micropump according to one of claims 7 and 8, characterized in that on the side opposed to said rib (16c), said membrane (16a) is provided with a boss (39; figure 6) extending towards another thin slab (28) of the pump and designed for limiting the motion of the membrane (16), should the same be exposed to an excessive pressure.

10. A micropump according to any one of claims 7 to 9, characterized in that the ribs (16c) provided respectively on the membranes (16a) of said valves are provided on opposite faces of these membranes (valves 45 and 46, figures 8, 9 and 10).

11. A micropump according to any one of the preceding claims, characterized in that the inlet (2; 30, 50) of the pump is in communication with a compensation chamber (21; 30; 51; 54) formed above said second valve (18; 36; 46), to bias the latter to close under the effect of the pressure prevailing at said inlet.

## Patentansprüche

1. Mikropumpe mit einem ersten Plättchen (11; 27; 41; 56) aus einem Werkstoff, der mit photolithographischen Methoden so bearbeitet werden kann, dass das Plättchen zusammen mit einem zweiten Plättchen (1; 26; 40), mit dem es flächig verbunden ist und das als Auflageplättchen dient, eine Pumpenkammer (15; 44) bildet, einem ersten Ventil des Rückschlagtyps (16; 33; 45), durch das die benannte Pumpenkammer (15; 44) wahlweise mit einem Pumpeneinlass (2; 30; 50) verbunden werden kann, und einem zweiten Ventil des Membrantyps (18; 36; 46), durch das die benannte Pumpenkammer wahlweise mit einem Pumpenaustritt (3; 36*a*; 43) verbunden werden kann, wobei Organe (13; 14; 29; 49) vorgesehen sind, um eine periodische Veränderung des Volumens der benannten Pumpenkammer (15; 44) hervorzurufen, und das zweite Ventil durch den Druck des aus der Pumpe zu fördernden Fluidums gesteuert wird, dadurch gekennzeichnet, dass der benannte Austritt (3; 36*a*; 43) unmittelbar mit einem Raum (18*d*) in Verbindung steht, der von der benannten Pumpenkammer (15; 44) durch das benannte zweite Ventil (18; 36; 46) getrennt und auf der gleichen Seite dieses Ventils angeordnet ist wie der Kanal (19; 20), über den dieses Ventil mit der Pumpenkammer in Verbindung steht, so dass die Drücke, die jeweils in dieser Pumpenkammer und im Austrittskanal herrschen, öffnend auf das benannte zweite Ventil wirken, und dass das benannte zweite Ventil (18; 36; 46) über die benannte Pumpenkammer (15; 44) in offener Verbindung mit dem benannten ersten Ventil (16; 33; 45) steht, so dass in der Förderphase der Pumpe diese Kammer über das benannte, in geöffneter Stellung befindliche zweite Ventil in direkter Verbindung mit dem benannten Austritt (3; 36*a*; 43) steht.

2. Mikropumpe gemäss Anspruch 1, dadurch gekennzeichnet, dass sie ein drittes Plättchen (12; 28; 42; 55) umfasst, das das benannte erste Plättchen (11; 27; 41) zumindest auf einem Teil seiner Oberfläche überdeckt und mit ihm zusammen zumindest eine Kammer (15; 21; 51; 54) begrenzt, die dazu bestimmt ist, einen aktiven Pumpraum zu bilden.

3. Mikropumpe gemäss Anspruch 2, dadurch gekennzeichnet, dass die benannten Organe, die dafür vorgesehen sind, eine periodische Veränderung des Pumpenkammervolumens zu bewirken, ein piezoelektrisches Plättchen (13; 29) umfassen, das auf dem benannten dritten Plättchen (12; 28) in einer Zone befestigt ist, die mit der benannten Pumpkammer (15) zusammenfällt.

4. Mikropumpe gemäss Anspruch 2, dadurch gekennzeichnet, dass die benannten Organe, die dafür vorgesehen sind, eine periodische Veränderung des Pumpenkammervolumens zu bewirken, ein piezoelektrisches Plättchen (49) umfassen, dass die benannte Pumpenkammer (44) vom benannten ersten (41) und zweiten (40) Plättchen begrenzt wird und dass das benannte piezoelektrische Plättchen auf dem benannten ersten Plättchen in einer unbedeckten Zone befestigt ist, die mit der benannten Pumpenkammer (44) zusammenfällt.

5. Mikropumpe gemäss einem beliebigen der Ansprüche 2 bis 4, dadurch gekennzeichnet, dass das benannte dritte Plättchen (55) dafür eingerichtet ist, gleichzeitig zumindest eine der Verbindungen zu bilden, vermittels derer die Pumpe an einen äusseren Kreis angeschlossen werden kann (Figur 11).

6. Mikropumpe gemäss einem beliebigen der Ansprüche 1 bis 5, dadurch gekennzeichnet, dass ein Sensorelement, zum Beispiel ein Dehnungsmesser (11*A*), auf dem benannten ersten Plättchen (11) befestigt ist, um die Pumpenfunktion zu überwachen.

7. Mikropumpe gemäss einem beliebigen der vorangehenden Ansprüche, dadurch gekennzeichnet, dass das benannte erste Ventil (16; 33; 45) und/oder das benannte zweite Ventil (18; 36; 46) in der Ebene des benannten ersten Plättchens ein Membrane (16*a*; 18*a*) aufweisen, die in der Mitte eine ringförmige Rippe (16*c*; 18*c*) trägt, deren Scheitel dazu bestimmt ist, gegen ein anderes Plättchen (1; 12; 26; 28; 40; 41) angedrückt zu werden, das als Ventilsitz dient, und dass zumindest die benannte Rippe (16*c*; 18*c*) von einer Oxidschicht (17) bedeckt ist, die der Membran eine vorgegebene Vorspannung aufgibt, so dass das Ventil normalerweise geschlossen ist.

8. Mikropumpe zufolge des Anspruchs 7, dadurch gekennzeichnet, dass die Membran (18*a*) des zweiten Ventils zumindest teilweise von einer Materialschicht bedeckt ist, die in der Membran Scherkräfte hervorruft, die eine zusätzliche Vorspannung induzieren.

9. Mikropumpe zufolge eines beliebigen der Ansprüche 7 und 8, dadurch gekennzeichnet, dass auf der der benannten Rippe (16*c*) entgegengesetzten Seite die benannte Membran (16*a*) mit einem Vorsprung (39; Figur 6) versehen ist, der sich in Richtung auf ein anderes Plättchen (28) erstreckt und dazu bestimmt ist, die Ausbiegung der Membran (16) in solchen Fällen zu begrenzen, in denen sie einem übermässigen Druck ausgesetzt ist.

10. Mikropumpe zufolge eines beliebigen der Ansprüche 7 bis 9, dadurch gekennzeichnet, dass die Rippen (16*c*), die jeweils auf den Membranen (16*a*) der benannten Ventile vorgesehen sind, auf entgegengesetzten Seiten dieser Membranen aufgebracht sind (Ventile 45 und 46, Figuren 8, 9 und 10).

11. Mikropumpe zufolge eines beliebigen der vorangehenden Ansprüche, dadurch gekennzeichnet, dass der Pumpeneinlass (2; 30; 50) mit einer Ausgleichskammer (21; 30; 51; 54) in Verbindung steht, die oberhalb des benannten zweiten Ventils (18; 36; 46) angebracht ist, um unter dem Einfluss des im benannten Einlass herrschenden Drucks dieses Ventil zur Schliessung zu veranlassen.
